# EUROPEAN PATENT APPLICATION

(11) **EP 4 581 942 A1**
(43) Date of publication of application: **09.07.2025**
(21) Application number: 23860355.9
(22) Date of filing: 29.08.2023
(51) Int. Cl.: A23L 33/125, A61K 31/70, A61K 31/7004, A61K 31/7016, A61K 31/702, A61K 31/715, A61P 1/04, A61P 1/10, A61P 43/00

(54) **COMPOSITION FOR IMPROVING NICOTINAMIDE MONONUCLEOTIDE PRODUCTION**

(30) Priority: 30.08.2022 JP 2022136945
(71) Applicant: Meiji Holdings Co., Ltd., Tokyo 104-0031 (JP)
(72) Inventor: OZAKI Satoru, Hachioji-shi, Tokyo 192-0919 (JP); HONME Yoshiko, Hachioji-shi, Tokyo 192-0919 (JP); WAKABAYASHI Jun, Hachioji-shi, Tokyo 192-0919 (JP); SAITO Yoshie, Hachioji-shi, Tokyo 192-0919 (JP); HIGASHI Seiichirou, Hachioji-shi, Tokyo 192-0919 (JP); MORIFUJI Masashi, Hachioji-shi, Tokyo 192-0919 (JP)
(74) Representative: TBK
(86) International application number: PCT/JP2023/031257
(87) International publication number: WO 2024/048593

(57) **Abstract**

A composition for improving nicotinamide mononucleotide (NMN) production, comprising at least one carbohydrate selected from the group consisting of monosaccharides (excluding glucose and xylose), disaccharides, trisaccharides, tetrasaccharides, pentasaccharides, sugar alcohols, and dietary fiber as an active ingredient.

## Description

### [Technical Field]

The present invention relates to a composition for improving nicotinamide mononucleotide production, and more specifically, to a composition for improving the production of nicotinamide mononucleotide by the gut microbiota.

### [Background Art]

Nicotinamide mononucleotide (NMN) is a precursor of nicotinamide adenine dinucleotide (NAD) and a substance that has attracted particular attention in recent years.

For example, maintaining the activity of sirtuins, known as longevity genes, is considered important for controlling aging; however, a decrease in the tissue concentration of NAD, which is necessary for their activity, with age is considered to be a cause of aging, and therefore, administration or ingestion of NMN, a precursor of NAD, is said to have anti-aging effects.

More specifically, for example, Mills Kathryn F. et al., Cell Metabolism, 24(6), 2016, pp. 795-806 (NPL 1) reports that administration of NMN to aged mice suppressed age-related obesity, improved energy metabolism, and improved insulin resistance, lipid metabolism, eye function, bone density, and immune function; this study also observed the suppression of age-related changes in gene expression in skeletal muscle, fat, and liver. Further, type II diabetes is a lifestyle-related disease caused by a decline in glucose tolerance with age, and Jun Yoshino et al., Cell Metab., 2011 Oct 5, 14 (4), pp. 528-536 (NPL 2) reports that administration of NMN improved glucose tolerance disorder and hepatic insulin sensitivity, and restored gene expression related to oxidative stress, inflammatory response, and circadian rhythm in mice with type II diabetes induced by a high-fat diet.

In addition, for example, Luis Rajman et al., Cell Metab., 2018 Mar 6, 27(3), pp. 529-547 (NPL 3) and Xiaonan Wang et al., Brain Research, Volume 1643, 15 July 2016, pp. 1-9 (NPL 4) report that NMN administration improved age-related declines in liver function, renal function, skeletal muscle function, and cardiac function, as well as dementia of the Alzheimer type. Furthermore, for example, Zhu et al., Signal Transduction and Targeted Therapy, 2017, e17017 (NPL 5) reports that NMN administration to aged mice improved constipation.

Therefore, in recent years, supplements and the like for ingesting NMN have been commercially available. However, a method for easily producing industrially sufficient amounts of NMN has not yet been established, and because the raw materials are difficult to obtain, there has been a problem that such supplements are expensive for daily intake.

As methods for producing NMN, in addition to production by chemical synthesis, methods using nicotinamide riboside (NR) as a raw material, methods using *Escherichia coli,* methods using yeast, and the like are known. It has also been reported that E. *coli* produces NMN from glucose and xylose (S. Shoji et al, Metabolic Engineering, 65, 2021, pp. 167-177 (NPL 6); Y. Liu et al., Microbial biotechnology, 2021, 14(6), pp. 2581-2591 (NPL 7); U. Ngivprom, ChemBioChem, 2022, 23, e202200071 (NPL 8)), and that NMN-containing yeast extract is produced in a glucose-containing medium (International Publication No. WO2017/022768 (PTL 1)). Furthermore, it has been reported that certain species of lactic acid bacteria also produce NMN (Sugiyama Kazane et al., Scientific Reports, 11(1), 2021, pp. 1-8 (NPL 9)). Moreover, for example, International Application Japanese-Phase Publication No. 2022-505262 (PTL 2) describes a pet food composition containing protein, carbohydrate, insoluble fiber, and soluble fiber as a composition that increases the level of oxidized NAD (NAD⁺) measured in fecal samples.

### [Citation List]

### [Patent Literature]

[PTL 1] International Publication No. WO2017/022768
[PTL 2] International Application Japanese-Phase Publication No. 2022-505262

### [Non Patent Literature]

[NPL 1] Mills Kathryn F. et al., Cell Metabolism, 24 (6), 2016, pp. 795-806
[NPL 2] Jun Yoshino et al., Cell Metab., 2011 Oct 5, 14 (4), pp. 528-536
[NPL 3] Luis Rajman et al., Cell Metab., 2018 Mar 6, 27(3), pp. 529-547
[NPL 4] Xiaonan Wang et al., Brain Research, Volume 1643, 15 July 2016, pp. 1-9
[NPL 5] Zhu et al., Signal Transduction and Targeted Therapy, 2017, e17017
[NPL 6] S. Shoji et al, Metabolic Engineering, 65, 2021, pp. 167-177
[NPL 7] Y. Liu et al., Microbial biotechnology, 2021, 14(6), pp. 2581-2591
[NPL 8] U. Ngivprom, ChemBioChem, 2022, 23, e202200071
[NPL 9] Sugiyama Kazane et al., Scientific Reports, 11(1), 2021, pp. 1-8

### [Summary of Invention]

### [Technical Problem]

Numerous bacteria inhabit the intestines (especially the large intestine (colon)) of animals, including humans, and the collection of these bacteria is called the gut microbiota. The gut microbiota is thought to play various roles, such as assisting digestion, regulating immune responses, and having anti-inflammatory effects; it is also known that the host absorbs metabolites produced by the gut microbiota, which brings various health benefits to the host. In view of the problems of the related art, the present inventors surmised that when the gut microbiota produces NMN and the host absorbs it, it would be possible to produce the same effects as when the host is administered or ingests NMN. The present invention has been made in view of such problems and findings, and an object of the present invention is to provide a composition having an effect of enhancing (improving) nicotinamide mononucleotide (NMN) production by the gut microbiota.

### [Solution to Problem]

The present inventors have conducted intensive studies to achieve the above object and first found that the gut microbiota produces NMN, although there are differences depending on individual differences in the host. Therefore, the present inventors searched for prebiotics capable of improving the amount of NMN produced by the gut microbiota from various conventionally known prebiotics (i.e., food components that are not decomposed or absorbed in the upper digestive tract, serve as selective nutrients for intestinal bacteria in the large intestine, improve and maintain the composition of the gut microbiota in a healthy balance, and help promote and maintain the health of the host (Gibson et al., J Nutr., 1995, 125, pp. 1401-1412, etc.)). As a result, the present inventors have newly found that at least one carbohydrate selected from the group consisting of specific carbohydrates, namely monosaccharides (excluding glucose and xylose), disaccharides, trisaccharides, tetrasaccharides, pentasaccharides, sugar alcohols, and dietary fiber, has the effect of increasing the amount of NMN produced by the gut microbiota both *in vitro* and *in vivo.* Thus, it has been clarified that by administering or ingesting a composition comprising the carbohydrate to a host, the amount of NMN produced by the gut microbiota can be increased. It has also been clarified that this allows the host to ingest more NMN via the large intestine, and thus, in addition to the NMN, the *in vivo* concentration of NAD, for which NMN is a precursor, and its metabolites can be increased. Furthermore, since ingestion of NMN can improve age-related diseases (NPLs 1 to 4) and improve constipation (NPL 5), and NAD, a metabolite of NMN, is effective in improving inflammatory bowel disease (Navarro et al., Br J Pharmacol., 2022, 179, pp. 1839-1856), it has also been clarified that administration or ingestion of a composition comprising the carbohydrate as an active ingredient can prevent or ameliorate age-related diseases, and prevent or ameliorate constipation and inflammatory bowel disease, thereby completing the present invention. The aspects of the present invention obtained by these findings are as follows.
[1] A composition for improving nicotinamide mononucleotide (NMN) production, comprising at least one carbohydrate selected from the group consisting of monosaccharides (excluding glucose and xylose), disaccharides, trisaccharides, tetrasaccharides, pentasaccharides, sugar alcohols, and dietary fiber as an active ingredient.
[2] The composition according to [1], comprising a monosaccharide as the carbohydrate, wherein the monosaccharide is tagatose.
[3] The composition according to [1] or [2], comprising a disaccharide as the carbohydrate, wherein the disaccharide is at least one selected from the group consisting of cellobiose, lactulose, trehalose, and sialyllactose.
[4] The composition according to any one of [1] to [3], comprising a trisaccharide as the carbohydrate, wherein the trisaccharide is at least one selected from the group consisting of kestose, lactosucrose, raffinose, and fucosyllactose.
[5] The composition according to any one of [1] to [4], comprising a tetrasaccharide as the carbohydrate, wherein the tetrasaccharide is at least one selected from the group consisting of stachyose and nystose.
[6] The composition according to any one of [1] to [5], comprising a pentasaccharide as the carbohydrate, wherein the pentasaccharide is fructofuranosylnystose.
[7] The composition according to any one of [1] to [6], comprising a sugar alcohol as the carbohydrate, wherein the sugar alcohol is at least one selected from the group consisting of reduced isomaltulose, lactitol, sorbitol, mannitol, and xylitol.
[8] The composition according to any one of [1] to [7], comprising dietary fiber as the carbohydrate, wherein the dietary fiber is inulin.
[9] The composition according to any one of [1] to [8], which is an oral composition or an enteral composition.
[10] The composition according to any one of [1] to [9], which is a food and/or drink composition, a pharmaceutical composition, or a quasi-pharmaceutical composition.
[11] The composition according to any one of [1] to [10], which is a composition for improving a concentration of nicotinamide adenine dinucleotide (NAD) *in vivo.*
[12] The composition according to any one of [1] to [10], which is a composition for preventing or ameliorating constipation.
[13] The composition according to [11] or [12], wherein the dose to humans is 10 to 1000 mg of the carbohydrate per kg body weight per day.
[14] The composition according to any one of [1] to [10], which is a composition for preventing or ameliorating inflammatory bowel disease.
[15] The composition according to [14], wherein the dose to humans is 10 to 1000 mg of the carbohydrate per kg body weight per day.
[16] The composition according to any one of [1] to [10], which is a composition for preventing or ameliorating age-related diseases.
[17] The composition according to [16], wherein the dose to humans is 10 to 1000 mg of the carbohydrate per kg body weight per day.
[18] Use of at least one carbohydrate selected from the group consisting of monosaccharides (excluding glucose and xylose), disaccharides, trisaccharides, tetrasaccharides, pentasaccharides, sugar alcohols, and dietary fiber for improving NMN production.
[19] Use of at least one carbohydrate selected from the group consisting of monosaccharides (excluding glucose and xylose), disaccharides, trisaccharides, tetrasaccharides, pentasaccharides, sugar alcohols, and dietary fiber for the production of a composition for improving NMN production.
[20] A method for improving NMN production, comprising administering to a subject an effective amount of at least one carbohydrate selected from the group consisting of monosaccharides (excluding glucose and xylose), disaccharides, trisaccharides, tetrasaccharides, pentasaccharides, sugar alcohols, and dietary fiber.
[21] The use of the carbohydrate according to [18], further for improving in *vivo* NAD concentration.
[22] The use of the carbohydrate according to [19], wherein the composition for improving NMN production is a composition for improving *in vivo* NAD concentration.
[23] The method for improving NMN production according to [20], which is further a method for improving in *vivo* NAD concentration.
[24] The use of the carbohydrate according to [18], further for preventing or ameliorating constipation.
[25] The use of the carbohydrate according to [19], wherein the composition for improving NMN production is a composition for preventing or ameliorating constipation.
[26] The method for improving NMN production according to [20], which is further a method for preventing or ameliorating constipation.
[27] The use of the carbohydrate according to [18], further for preventing or ameliorating inflammatory bowel disease.
[28] The use of the carbohydrate according to [19], wherein the composition for improving NMN production is a composition for preventing or ameliorating inflammatory bowel disease.
[29] The method for improving NMN production according to [20], which is further a method for preventing or ameliorating inflammatory bowel disease.
[30] The use of the carbohydrate according to [18], further for preventing or ameliorating age-related diseases.
[31] The use of the carbohydrate according to [19], wherein the composition for improving NMN production is a composition for preventing or ameliorating age-related diseases.
[32] The method for improving NMN production according to [20], which is further a method for preventing or ameliorating age-related diseases.

### [Advantageous Effects of Invention]

According to the present invention, it is possible to provide a composition having an effect of enhancing (improving) nicotinamide mononucleotide (NMN) production by the gut microbiota. Further, the composition of the present invention can be provided as a food and/or drink composition, and therefore, it is easy to ingest and easy to make ingestion a habit. Furthermore, the composition of the present invention can be used for enhancing (improving) NAD concentration *in vivo,* preventing or ameliorating age-related diseases, and preventing or ameliorating constipation and inflammatory bowel disease by increasing the amount of NMN produced by the gut microbiota.

### [Brief Description of Drawings]

[Fig. 1] Fig. 1 is a graph showing the NMN concentration in cultures of human feces (#1 to 3) cultured in GAM medium comprising 0.5% glucose obtained in Test Example 1.
[Fig. 2] Fig. 2 is a graph showing the average values of NMN concentrations in cultures of human feces (#1 to 3) cultured in evaluation media comprising each carbohydrate obtained in Test Example 2.
[Fig. 3] Fig. 3 is a graph showing the average values of the amount of NMN in cecal contents (the amount (total) obtained by multiplying the NMN concentration by the weight of cecal contents) in the Control group and the FOS group obtained in Test Example 3.
[Fig. 4] Fig. 4 is a graph showing the average values of NMN concentrations in feces of human flora mice on each fecal collection day obtained in Test Example 4.

### [Description of Embodiments]

Hereinafter, the present invention will be described in detail based on its preferred embodiments.

### <Composition for Improving NMN Production>

The present invention provides a composition for improving (enhancing) nicotinamide mononucleotide (NMN) production (sometimes referred to as "composition for improving NMN production" or simply "composition of the present invention" in the present specification), comprising, as an active ingredient, at least one carbohydrate selected from the group consisting of monosaccharides (excluding glucose and xylose), disaccharides, trisaccharides, tetrasaccharides, pentasaccharides, sugar alcohols, and dietary fiber.

In the present invention, nicotinamide mononucleotide refers to β-nicotinamide mononucleotide, which is also abbreviated as NMN. β-Nicotinamide mononucleotide is an intermediate metabolite when nicotinamide and nicotinamide riboside are converted to nicotinamide adenine dinucleotide (NADH, NAD⁺, sometimes abbreviated as "NAD " in the present specification) *in vivo.* Nicotinamide adenine dinucleotide is known to play a central role in *in vivo* dehydrogenation reactions as an oxidation-reduction coenzyme. The present inventors have found that nicotinamide mononucleotide is produced by the gut microbiota, and that the carbohydrates according to the present invention increase the production amount of such nicotinamide mononucleotide and have an excellent effect of improving nicotinamide mononucleotide production.

In the present invention, gut microbiota refers to a population of bacteria present in the intestinal tract (especially the large intestine) of animals, and is also referred to as intestinal flora or gut microbiome. Microorganisms included in the gut microbiota according to the present invention include, in addition to intestinal bacteria that make up the majority, other intestinal microorganisms (phages, viruses, archaea, fungi, parasites). For example, it is generally said that more than 1000 types of intestinal bacteria exist in the large intestine of humans, in the amount of 600 to 1000 trillion. The composition of the gut microbiota varies greatly among individuals and also changes with the age and health status of the host. Therefore, although it cannot be stated generally, for example, a group of microorganisms obtained by culturing human or animal feces in a GAM medium comprising 0.5% glucose at 37°C under anaerobic conditions for 12 hours may be used as the gut microbiota according to the present invention.

The active ingredient comprised in the composition for improving NMN production of the present invention is at least one carbohydrate selected from the group consisting of monosaccharides (excluding glucose and xylose), disaccharides, trisaccharides, tetrasaccharides, pentasaccharides, sugar alcohols, and dietary fiber. The carbohydrate according to the present invention is preferably a saccharide, more preferably a sugar, still more preferably at least one selected from the group consisting of disaccharides, trisaccharides, tetrasaccharides, and pentasaccharides, even more preferably at least one selected from the group consisting of trisaccharides and tetrasaccharides, and particularly preferably a trisaccharide and then a tetrasaccharide. The carbohydrate according to the present invention is preferably one that does not contain fucose as a constituent sugar, and more preferably one that comprises 1 to 3 types selected from the group consisting of glucose, galactose, and fructose as constituent sugars.

### (Monosaccharides)

The monosaccharide according to the present invention may be linear or cyclic, may be an aldose comprising an aldehyde group or a ketose comprising a ketone group, and when both D-form and L-form exist, it may be either of them. The number of carbon atoms in the monosaccharide according to the present invention is preferably 4 to 7, more preferably 6 (i.e., the monosaccharide is a hexose).

However, glucose and xylose are excluded from the monosaccharides according to the present invention. Glucose and xylose are completely absorbed into the body in the small intestine and therefore do not reach the large intestine (Yamada Kazuhiko et al., Japanese Journal of Nutrition and Dietetics, 2001, Vol. 59, No. 4, pp. 169-176; Keio University Hospital, ''D-Xylose Test", [online], February 22, 2018, [searched on August 3, 2023], Internet <https://kompas.hosp.keio.ac.jp/contents/000347.html>), and do not contribute to improving NMN production by intestinal bacteria present in the large intestine.

More specific examples of the monosaccharide according to the present invention include tagatose, arabinose, allose, psicose, galactose, fructose, and mannose, which may be used alone or in combination of two or more. Among them, the monosaccharide according to the present invention is preferably at least one selected from the group consisting of tagatose, arabinose, allose, and psicose, and more preferably tagatose from the viewpoint of being easily reachable by intestinal bacteria that produce NMN *in vivo,* that is, easily reaching the large intestine without being decomposed or absorbed in the upper digestive tract as a prebiotic.

### (Disaccharides)

The disaccharide according to the present invention refers to a sugar in which two molecules of the monosaccharide are bonded by dehydration condensation. When both D-form and L-form exist, it may be either of them. The disaccharide according to the present invention also includes a modified sugar in which the disaccharide is modified. Examples of the modification of the disaccharide include sialylation, and the modification site may be one or more, and when there are multiple sites, it may be one type of modification or a combination of two or more types of modifications.

The monosaccharides constituting the disaccharide may be the same as or different from each other, and examples thereof include tagatose, glucose, galactose, fructose, mannose, xylose, arabinose, allose, and psicose, with tagatose, glucose, galactose, fructose, mannose, and xylose being preferred. Among them, the disaccharide according to the present invention is preferably a disaccharide comprising, as constituent sugars, one or two selected from the group consisting of glucose, galactose, and fructose.

More specific examples of the disaccharide according to the present invention include cellobiose, lactulose, trehalose, lactose, and isomaltulose, which may be used alone or in combination of two. Among them, the disaccharide according to the present invention is preferably at least one selected from the group consisting of cellobiose, lactulose, trehalose, and sialyllactose (such as 3'-sialyllactose) from the viewpoint of being easily reachable by intestinal bacteria that produce NMN *in vivo,* that is, easily reaching the large intestine without being decomposed or absorbed in the upper digestive tract as a prebiotic, more preferably a disaccharide that is not modified, still more preferably at least one selected from the group consisting of cellobiose, lactulose, and trehalose, and particularly preferably cellobiose.

### (Trisaccharides)

The trisaccharide according to the present invention refers to a sugar in which three molecules of the monosaccharide are bonded by dehydration condensation. When both D-form and L-form exist, it may be either of them. The trisaccharide according to the present invention also includes a modified sugar in which the trisaccharide is modified. Examples of the modification of the trisaccharide are the same as those of the disaccharide, and the modification site may be one or more, and when there are multiple sites, it may be one type of modification or a combination of two or more types of modifications.

The monosaccharides constituting the trisaccharide may all be the same, some may be the same, or all may be different, and examples thereof include tagatose, glucose, galactose, fructose, mannose, xylose, arabinose, allose, psicose, and fucose, with tagatose, glucose, galactose, fructose, mannose, xylose, and fucose being preferred. Among them, the trisaccharide according to the present invention is preferably a trisaccharide comprising, as constituent sugars, 1 to 3 types selected from the group consisting of glucose, galactose, fructose, and fucose, more preferably a trisaccharide comprising, as constituent sugars, 1 to 3 types selected from the group consisting of glucose, galactose, and fructose.

More specific examples of the trisaccharide according to the present invention include kestose (such as 1-kestose), lactosucrose, raffinose, fucosyllactose (such as 2'-fucosyllactose), and galactosyllactose (such as 4'-galactosyllactose), which may be used alone or in combination of two or more. Among them, the trisaccharide according to the present invention is preferably at least one selected from the group consisting of kestose, lactosucrose, raffinose, fucosyllactose, and galactosyllactose from the viewpoint of being easily reachable by intestinal bacteria that produce NMN *in vivo,* that is, easily reaching the large intestine without being decomposed or absorbed in the upper digestive tract as a prebiotic, more preferably at least one selected from the group consisting of kestose, lactosucrose, and raffinose.

### (Tetrasaccharides)

The tetrasaccharide according to the present invention refers to a sugar in which four molecules of the monosaccharide are bonded by dehydration condensation. When both D-form and L-form exist, it may be either of them. The tetrasaccharide according to the present invention also includes a modified sugar in which the tetrasaccharide is modified. Examples of the modification of the tetrasaccharide are the same as those of the disaccharide, and the modification site may be one or more, and when there are multiple sites, it may be one type of modification or a combination of two or more types of modifications.

The monosaccharides constituting the tetrasaccharide may all be the same, some may be the same, or all may be different, and examples thereof include tagatose, glucose, galactose, fructose, mannose, xylose, arabinose, allose, and psicose, with tagatose, glucose, galactose, fructose, mannose, and xylose being preferred. Among them, the tetrasaccharide according to the present invention is preferably a tetrasaccharide comprising, as constituent sugars, 1 to 3 types selected from the group consisting of glucose, galactose, and fructose.

The tetrasaccharide according to the present invention may be used alone or in combination of two or more. Among them, the tetrasaccharide according to the present invention is preferably at least one selected from the group consisting of stachyose and nystose from the viewpoint of being easily reachable by intestinal bacteria that produce NMN *in vivo,* that is, easily reaching the large intestine without being decomposed or absorbed in the upper digestive tract as a prebiotic.

### (Pentasaccharides)

The pentasaccharide according to the present invention refers to a sugar in which five molecules of the monosaccharide are bonded by dehydration condensation. When both D-form and L-form exist, it may be either of them. The pentasaccharide according to the present invention also includes a modified sugar in which the pentasaccharide is modified. Examples of the modification of the pentasaccharide are the same as those of the disaccharide, and the modification site may be one or more, and when there are multiple sites, it may be one type of modification or a combination of two or more types of modifications.

The monosaccharides constituting the pentasaccharide may all be the same, some may be the same, or all may be different, and examples thereof include tagatose, glucose, galactose, fructose, mannose, xylose, arabinose, allose, and psicose. Among them, the pentasaccharide according to the present invention is preferably a pentasaccharide comprising, as constituent sugars, 1 to 3 types selected from the group consisting of glucose, galactose, and fructose.

The pentasaccharide according to the present invention may be used alone or in combination of two or more. Among them, the pentasaccharide according to the present invention is preferably fructofuranosylnystose (such as 1-fructofuranosyl-D-nystose) from the viewpoint of being easily reachable by intestinal bacteria that produce NMN *in vivo,* that is, easily reaching the large intestine without being decomposed or absorbed in the upper digestive tract as a prebiotic.

### (Sugar Alcohols)

The sugar alcohol according to the present invention is a compound in which the carbonyl group of a sugar compound is reduced. Examples of the sugar compound include the monosaccharide, disaccharide, trisaccharide, tetrasaccharide, and pentasaccharide.

More specific examples of the sugar alcohol according to the present invention include lactitol, sorbitol, mannitol, xylitol, and reduced isomaltulose, which may be used alone or in combination of two or more. Among them, the sugar alcohol according to the present invention is preferably a sugar alcohol formed by reducing the monosaccharide or disaccharide, more preferably at least one selected from the group consisting of lactitol, sorbitol, mannitol, xylitol, and reduced isomaltulose from the viewpoint of being easily reachable by intestinal bacteria that produce NMN *in vivo,* that is, easily reaching the large intestine without being decomposed or absorbed in the upper digestive tract as a prebiotic, and still more preferably at least one selected from the group consisting of lactitol and sorbitol.

### (Dietary Fiber)

The dietary fiber according to the present invention is a carbohydrate that is not decomposed by human digestive enzymes (such as amylase, protease, and maltase), and refers to a sugar in which six or more monosaccharide molecules are bonded by dehydration condensation. The dietary fiber may be linear or branched. The molecular weight of the dietary fiber according to the present invention is preferably 500 to 50000, more preferably 1000 to 30000.

The monosaccharides constituting the dietary fiber may all be the same, some may be the same, or all may be different, and examples thereof include tagatose, glucose, galactose, fructose, mannose, xylose, arabinose, allose, and psicose. Among them, the dietary fiber according to the present invention is preferably a dietary fiber comprising, as constituent sugars, 1 to 3 types selected from the group consisting of glucose, galactose, and fructose.

More specific examples of the dietary fiber according to the present invention include inulin, indigestible dextrin, and guar gum degradation products, which may be used alone or in combination of two or more. Among them, the dietary fiber according to the present invention is preferably inulin.

The carbohydrate according to the present invention may be a combination of two or more selected from the group consisting of monosaccharides, disaccharides, trisaccharides, tetrasaccharides, pentasaccharides, sugar alcohols, and dietary fiber, as described above. In this case, the combination of carbohydrates according to the present invention is not particularly limited, but is more preferably a combination of two or more saccharides selected from the group consisting of disaccharides, trisaccharides, tetrasaccharides, and pentasaccharides, more preferably trisaccharides, tetrasaccharides, and pentasaccharides (sometimes referred to as "mixed sugar" in the present specification). Examples of such combinations include fructooligosaccharides including kestose (trisaccharide), nystose (tetrasaccharide), and fructofuranosylnystose (pentasaccharide); galactooligosaccharides including galactosyllactose (trisaccharide); soybean oligosaccharides including raffinose (trisaccharide) and stachyose (tetrasaccharide); lactosucrose (trisaccharide); xylooligosaccharides including xylobiose (disaccharide); isomaltooligosaccharides including isomaltose (disaccharide), isomaltotriose (trisaccharide), and panose (trisaccharide); and coffee bean mannooligosaccharides which are a mixture of disaccharides to decasaccharides in which mannose is linearly β1,4-mannosidically bonded.

Among these, the mixed sugar according to the present invention is preferably at least one selected from the group consisting of fructooligosaccharides and galactooligosaccharides, more preferably fructooligosaccharides, from the viewpoint of being easily reachable by intestinal bacteria that produce NMN *in vivo,* that is, easily reaching the large intestine without being decomposed or absorbed in the upper digestive tract as a prebiotic.

In the composition of the present invention, the content of the carbohydrate (the total amount when the carbohydrate is a combination of two or more, the same applies hereinafter) is appropriately adjusted according to the form of the composition, the dose or intake, the purpose and method of administration or ingestion, etc., and is therefore not particularly limited, and can be 0.001 to 100% by mass relative to the total mass of the composition; for example, it can be in the range of 0.001 to 99% by mass, 0.005 to 95% by mass, 0.01 to 80% by mass, 0.1 to 70% by mass, 1 to 60% by mass, 5 to 50% by mass, or 10 to 50% by mass.

The dose or intake (hereinafter sometimes referred to as "dose") of the composition of the present invention can be appropriately determined on a case-by-case basis in consideration of the form of the composition; the purpose and method of administration or ingestion; the species, age, body weight, sex, and severity of symptoms of the subject, etc. Therefore, although not particularly limited, for example, the dose for humans (preferably adults) is preferably 1 to 1000 mg of the carbohydrate (the total amount when the carbohydrate is a combination of two or more, the same applies hereinafter) per kg body weight of the subject per day, more preferably 10 to 1000 mg, and for example, it can be 20 to 800 mg, 30 to 700 mg, or 50 to 500 mg.

The composition of the present invention may further comprise other food- or pharmaceutically-acceptable ingredients in addition to the carbohydrate, as long as the effects of the present invention are not inhibited. Examples of the other ingredients include, but are not particularly limited to, water, lipids, minerals, vitamins, proteins, peptides, amino acids, organic acids, lactic acid bacteria (such as *Lactobacillus* and *Bifidobacterium*), yeast, treated products of the lactic acid bacteria and/or yeast (such as crushed products and heat-treated products), fermented products of the lactic acid bacteria and/or yeast, and formulation auxiliaries. The other ingredients may also be sugars other than the carbohydrates or other active ingredients.

The lipid is not particularly limited, and more specific examples thereof include natural oils and fats such as soybean oil, corn oil, palm oil, perilla oil, canola oil, safflower oil, sunflower oil, sesame oil, rice oil, grape seed oil, fish oil, safflower oil, rapeseed oil, and peanut oil; and synthetic oils and fats such as medium-chain fatty acid triglycerides (MCT) having about 6 to 12 carbon atoms.

The minerals are not particularly limited, and more specific examples thereof include calcium, magnesium, sodium, potassium, phosphorus, iron, manganese, copper, zinc, iodine, zinc, selenium, chromium, and molybdenum.

The vitamins are not particularly limited, and more specific examples thereof include vitamins A, B1, B2, B5, B6, B7, B9, B12, C, D, E, and K.

The formulation auxiliaries are not particularly limited, and more specific examples thereof include solvents, dispersants, emulsifiers, thickeners, thickening stabilizers, gelling agents, surfactants, buffers, stabilizers, excipients, binders, disintegrants, lubricants, flavoring agents, solubilizers, suspending agents, coating agents, carriers (solid carriers, liquid carriers such as water), preservatives, fragrances, colorants, and pH adjusters.

The sugars other than the carbohydrates are not particularly limited, and more specific examples thereof include starch and maltodextrin.

The active ingredients other than the carbohydrates are not particularly limited, and more specific examples thereof include fruits, vegetables, and processed products thereof, animal and plant extracts, and naturally derived polymers (such as collagen, hyaluronic acid, and chondroitin).

The other ingredients may be comprised alone or in combination of two or more. When these other ingredients are comprised, the content of the other ingredients is not particularly limited and can be appropriately adjusted according to the form of the composition, the dose, the purpose and method of administration or ingestion, etc.

The administration form of the composition of the present invention is not particularly limited, and examples thereof include oral administration, enteral administration, and intraperitoneal administration, and the composition of the present invention can be a composition according to each administration form. Among them, the composition of the present invention is preferably administered or ingested orally or enterally, and is preferably an oral composition or an enteral composition accordingly. In particular, from the viewpoint of easiness, it is more preferable to administer or ingest orally, and more preferably to use an oral composition. Further, the composition of the present invention can be, for example, a pharmaceutical composition, a quasi-pharmaceutical composition, a food and/or drink composition, or a feed composition, depending on the purpose, subject, method, dose, etc. of administering or ingesting the composition.

The pharmaceutical composition and quasi-pharmaceutical composition according to the present invention can be, for example, a preparation, and the form thereof is not particularly limited, and examples thereof include solid preparations such as tablets, pills, granules, powders, fine powders, and capsules; liquid preparations such as general solutions, suspensions, emulsions, and syrups; jellies; enteral preparations; and external preparations such as suppositories. The preparation can be produced, for example, by adding one or more of the formulation auxiliaries to the carbohydrate, and further adding one or more of the other ingredients as necessary, by a known method or a method analogous thereto.

The form of the food and/or drink composition according to the present invention is not particularly limited, and examples thereof include solid forms such as bars, liquid forms such as beverages and liquid diets, pastes, semi-liquids, gels (jellies), gelled oils and fats (semi-solid oils and fats), and powders. The food and/or drink composition can also be ingested by patients receiving oral or enteral nutrition, elderly people, infants, etc. as liquid diets, powdered liquid diets, nutritional pastes, oral or tube-feeding nutrients, beverages, gelled foods, etc.

Examples of the food and/or drink composition according to the present invention include, but are not particularly limited to, beverages (tea, carbonated drinks, cocoa, coffee, lactic acid bacteria beverages, soy milk beverages, fruit/vegetable juice beverages, soft drinks, nutritional drinks, alcoholic beverages, etc.), processed foods (chocolate, gum, gummy candies, jelly, baked confectionery (bread, cakes, cookies, biscuits, etc.), candies, etc.), dairy products (prepared milk powder (powdered milk, etc.), modified milk, milk beverages, fermented milk, yogurt, ice cream, cheese, cream, butter, margarine, condensed milk, etc.), seasonings (sauces, soups, dressings, mayonnaise, mayonnaise-type seasonings, cream, etc.), supplements, edible oils, and functional edible oils and fats. Such a food and/or drink composition can be produced, for example, by a method of blending the carbohydrate according to the present invention with an existing food or drink, or a raw material or intermediate product in the production process thereof. The carbohydrate to be blended in this case may be in the form of the pharmaceutical composition or the quasi-pharmaceutical composition.

The food and/or drink composition according to the present invention may also be, for example, a general food, a health food, a functional food, a food with health claims (e.g., Foods for Specified Health Uses, foods with nutrient function claims, dietary supplements, foods with function claims, etc.), a food for special dietary uses (e.g., food for infants, food for pregnant and lactating women, food for the sick, etc.), a medical food (food prescribed under the supervision of a physician as defined by the U.S. Food and Drug Administration (FDA) and the Orphan Drug Act), a therapeutic diet (food that serves the purpose of treatment and is prepared based on a menu created by a dietitian, etc., according to a meal prescription by a physician), or a diet therapy food. Further, the food and/or drink composition may be labeled with the effects/efficacy brought about by the carbohydrate according to the present invention in the product (e.g., prevention or amelioration of age-related diseases, prevention or amelioration of constipation, or prevention or amelioration of inflammatory bowel disease).

Examples of the feed composition according to the present invention include those obtained by appropriately modifying the above food and/or drink composition according to the purpose, subject, method, dose, etc. of administering or ingesting the feed composition.

The composition of the present invention is preferably packaged (preferably enclosed) in a packaging container from after production until administration or ingestion. The packaging container is not particularly limited, and examples thereof include wrapping paper, packaging bags, soft bags, tubes, tear packs, paper containers, cans, bottles, and capsules.

By comprising the above-mentioned carbohydrate as an active ingredient, the composition of the present invention can be used for improving NMN production by the gut microbiota, more preferably improving NMN production by the gut microbiota *in vivo.* Therefore, the present invention also provides the use of the carbohydrate for improving NMN production, and the use of the carbohydrate for the production of a composition for improving NMN production.

Further, by improving NMN production by the gut microbiota and absorbing the produced NMN by the host (subject), it is possible to increase the NMN concentration *in vivo* and also increase the production of NAD, for which such NMN is a precursor, and further its metabolites (such as nicotinamide, nicotinic acid, NAMN, and NAAD), thereby increasing the *in vivo* concentrations. Therefore, the composition of the present invention can be used for improving NAD concentration *in vivo,* and the present invention also provides the use of the carbohydrate for improving *in vivo* NAD concentration, and the use of the carbohydrate for the production of a composition for improving in *vivo* NAD concentration.

Furthermore, the composition of the present invention can also be used for the prevention or amelioration of various diseases and symptoms that are reported to be prevented or ameliorated by administration or ingestion of NMN or its metabolite NAD, by increasing the *in vivo* NMN concentration and/or NAD concentration, such as age-related diseases (NPLs 1 to 4]), constipation (NPL 5), and inflammatory bowel disease (Navarro et al., Br J Pharmacol., 2022, 179, pp. 1839-1856), and is preferably used for the prevention or amelioration of age-related diseases, for the prevention or amelioration of constipation, or for the prevention or amelioration of inflammatory bowel disease. The present invention also provides the use of the carbohydrate for the prevention or amelioration of these diseases, and the use of the carbohydrate for the production of a composition for preventing or ameliorating these diseases.

The age-related diseases include various diseases reported to be caused by aging (preferably 40 years or older, more preferably 50 years or older, still more preferably 60 years or older in humans), and examples thereof include obesity, decreased insulin resistance, decreased lipid metabolism ability, decreased eye function, decreased bone density, decreased immune function, decreased glucose tolerance, increased oxidative stress, inflammation, circadian rhythm disorders, decreased liver function, decreased renal function, decreased skeletal muscle function, decreased cardiac function, dementia of the Alzheimer type, and decreased central nervous system function.

In the present invention, the improvement of NMN production by the gut microbiota *in vivo* can be confirmed, for example, by collecting feces, measuring the NMN concentration in the feces, and confirming that the NMN concentration has increased relative to a subject before administration of the composition of the present invention or the carbohydrate according to the present invention, or a group to which the composition or the carbohydrate is not administered. The NMN concentration can be measured by a known method or a method analogous thereto as appropriate, for example, it can be measured by a method using a liquid chromatograph mass spectrometer (LC-MS/MS) described in the Examples below.

### <Method for Improving NMN Production>

By administering the above-mentioned carbohydrate to a subject, NMN production by the gut microbiota of the subject can be improved (enhanced). Further, thereby, *in vivo* NAD concentration can be improved (enhanced), and the various diseases and symptoms (preferably age-related diseases, constipation, and inflammatory bowel disease) can be prevented or ameliorated. Therefore, the present invention also provides a method for improving NMN production, a method for improving *in vivo* NAD concentration, and a method for preventing or ameliorating the various diseases (preferably age-related diseases, constipation, and inflammatory bowel disease) (hereinafter sometimes collectively referred to as "method of the present invention"), comprising administering an effective amount of the carbohydrate to a subject.

The method of the present invention includes a step of administering an effective amount of the carbohydrate according to the present invention to a subject. The subject according to the method of the present invention includes humans and non-human mammals, and examples of the non-human mammals include mice, sheep, cattle, pigs, horses, monkeys, dogs, cats, and rabbits. Since there are individual differences in the gut microbiota as described above, and some individuals may not produce NMN, the subject is preferably one whose gut microbiota produces NMN. Whether the gut microbiota produces NMN cannot be stated generally due to individual differences, but it can be determined, for example, by detecting NMN in a medium obtained by culturing feces (preferably 1 mg) of a subject in a GAM medium (preferably 1 mL) comprising 0.5% glucose at 37°C under anaerobic conditions for 12 hours (preferably, a value equal to or greater than the limit of quantitation (e.g., 1 ng/mL) is detected when measured by a method using a liquid chromatograph mass spectrometer (LC-MS/MS) (e.g., the method described in the Examples below)). The subject may be a person already suffering from the various diseases (e.g., age-related diseases, constipation, inflammatory bowel disease) or a person aiming to prevent the onset of the diseases.

In the method of the present invention, the carbohydrate can be administered as it is, more preferably as the composition of the present invention, and is preferably administered to the subject orally or enterally, more preferably orally. In the present invention, oral administration also includes ingestion of the food and/or drink composition, the feed composition, and the like.

In the method of the present invention, the effective amount of the carbohydrate is appropriately determined on a case-by-case basis in consideration of the purpose and method of administration; the species, age, body weight, sex, disease, and severity of symptoms of the subject, etc., and is therefore not particularly limited, but is, for example, the same as the dose of the composition of the present invention described above, that is, for humans (preferably adults), it is preferably 1 to 1000 mg of the carbohydrate per kg body weight of the subject per day, more preferably 10 to 1000 mg, and for example, it can be 20 to 800 mg, 30 to 700 mg, or 50 to 500 mg. In the method of the present invention, the frequency of administration can be once a day to an appropriate number of times per day.

### [Examples]

Hereinafter, the present invention will be described more specifically based on Test Examples, but the present invention is not limited to the following examples. In each of the Test Examples below, "%" indicates weight/volume (w/v: g/mL) percent unless otherwise specified.

### <Test Example 1> NMN Production Ability of Human Gut Microbiota

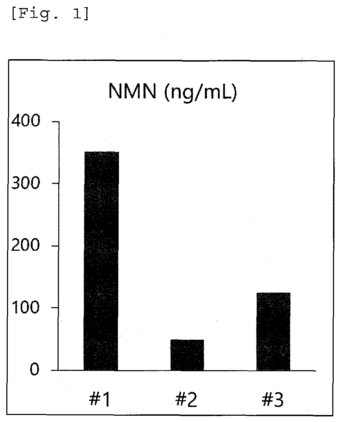

1 mg of human feces (#1 to 3) collected from different individuals was added to 1 mL of GAM medium supplemented with glucose so that the final concentration was 0.5%, and cultured at 37°C in an anaerobic glove box for 12 hours, and then the NMN concentration in the culture solution was measured. As the GAM medium, GAM semi-fluid medium for saccharolysis (manufactured by Nissui Pharmaceutical Co., Ltd.) was dissolved in water to a predetermined concentration, filtered through a 0.8 µm filter, and autoclaved at 115°C for 15 minutes. The NMN concentration was measured by the following method. That is, first, the supernatant obtained by centrifuging the culture solution was filtered through a 0.22 µm filter, and then the filtrate was mixed with methanol (final concentration: 80 v/v%) and allowed to stand on ice for 30 minutes. Next, the supernatant obtained by centrifuging this was dried using a centrifugal evaporator. Next, the obtained dried product was dissolved in an ammonium acetate solution and subjected to a liquid chromatograph mass spectrometer (LC-MS/MS) for analysis under the following conditions to measure the NMN concentration. The NMN concentrations (ng/mL) in each human feces (#1 to 3) are shown in Fig. 1.

### (LC-MS/MS analysis conditions)

Detector: QTRAP4500 (manufactured by SCIEX)
HPLC system: manufactured by Shimadzu Corporation
Column: Hypercarb 3 µm, 2.1 mm × 100 mm Column Mobile phase
   A solution: 7.5 mM ammonium acetate aqueous solution comprising 0.05 (v/v) % ammonium hydroxide
   B solution: acetonitrile solution comprising 0.05 (v/v) % ammonium hydroxide
   Ratio of solution A and solution B: Start of mobile phase flow → 0 min: 95 v/v% solution A - 5 v/v% solution B; 0 min → 1.8 min: 95 v/v% solution A - 5 v/v% solution B; 1.8 min → 14 min: 95 v/v% solution A - 5 v/v% solution B → 46 v/v% solution A - 54 v/v% solution B; 14 min → 14.1 min: 46 v/v% solution A - 54 v/v% solution B → 10 v/v% solution A - 90 v/v% solution B; 14.1 min → 17.1 min: 10 v/v% solution A - 90 v/v% solution B; 17.1 min → 17.2 min: 95 v/v% solution A - 5 v/v% solution B; 17.2 min → 32.2 min: 95 v/v% solution A - 5 v/v% solution B
Column temperature: 60°C
Flow rate: 0.2 mL/min
Injection volume: 2 µL
Ionization: ESI positive mode
m/z of NMN
   Precursor ion (Q1 Mass, (Da)): 335.1
   Product ion (Q3 Mass, (Da)): 123.0.

As shown in Fig. 1, NMN was detected in human feces #1 to #3, and it was confirmed that the human gut microbiota produces NMN. It was also confirmed that the human fecal culture system in the GAM medium can be used to evaluate the NMN production ability of the human fecal-derived gut microbiota.

### <Test Example 2> Evaluation of Improvement of NMN Production by Carbohydrates

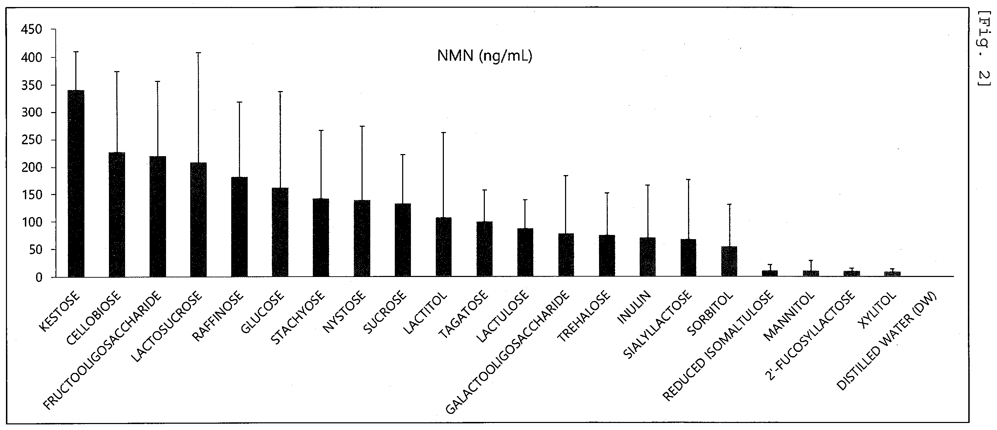

First, each evaluation medium was prepared by adding any one of the carbohydrates shown in Table 1 below to the GAM medium (without glucose added) so that the final concentration was 0.5%. As a comparative example, an evaluation medium to which distilled water (DW) was added instead of carbohydrate was also prepared. Next, 1 mg of the same human feces #1 to #3 as in Test Example 1 was added to 1 mL of the evaluation medium, and cultured at 37°C in an anaerobic glove box for 12 hours. After culturing, the NMN concentration in the culture solution was measured by liquid chromatograph mass spectrometer (LC-MS/MS) by the method described in Test Example 1. The average values of the NMN concentrations when human feces #1 to #3 were cultured are shown in Fig. 2. Table 2 below shows the results of calculating the average values for each carbohydrate classification (excluding glucose and sucrose) for the average values.

**[Table 1]**

| Carbohydrate | Substance Name | Manufacturer | Notes |
|---|---|---|---|
| Monosaccharide | Tagatose | Toronto Research Chemicals Inc. | D-Tagatose (Hexose) |
| | Glucose | FUJIFILM Wako Pure Chemical Corporation | D-Glucose (Hexose) |
| Disaccharide | Cellobiose | FUJIFILM Wako Pure Chemical Corporation | D-Cellobiose |
| | Lactulose | FUJIFILM Wako Pure Chemical Corporation | D-Lactulose |
| | Trehalose | FUJIFILM Wako Pure Chemical Corporation | D-Trehalose |
| | Sucrose | FUJIFILM Wako Pure Chemical Corporation | D-Sucrose |
| | Sialyllactose | Tokyo Chemical Industry Co., Ltd. | 3'-Sialyllactose |
| Trisaccharide | Kestose | Meiji Food Materia Co. Ltd | 1-Kestose |
| | Lactosucrose | FUJIFILM Wako Pure Chemical Corporation | Trade Name: Lactosyl Fructoside |
| | Raffinose | FUJIFILM Wako Pure Chemical Corporation | |
| | Fucosyllactose | Sigma Aldrich Co. LLC | 2'-Fucosyllactose |
| Tetrasaccharide | Stachyose | Tokyo Chemical Industry Co., Ltd. | |
| | Nystose | FUJIFILM Wako Pure Chemical Corporation | |
| Mixed Sugar | Fructooligosaccharide | Meiji Food Materia Co. Ltd | Trade Name: MeiOligo P |
| | Galactooligosaccharide | FUJIFILM Wako Pure Chemical Corporation | |
| Sugar Alcohol | Reduced Isomaltulose | FUJIFILM Wako Pure Chemical Corporation | Isomaltulose Reductant |
| | Lactitol | FUJIFILM Wako Pure Chemical Corporation | D-Lactitol |
| | Sorbitol | FUJIFILM Wako Pure Chemical Corporation | D-Sorbitol |
| | Mannitol | FUJIFILM Wako Pure Chemical Corporation | D-Mannitol |
| | Xylitol | FUJIFILM Wako Pure Chemical Corporation | D-Xylitol |
| Dietary Fiber | Inulin | Tokyo Chemical Industry Co., Ltd. | |

**[Table 2]**

| Carbohydrate | Substance Name | NMN Concentration (Avg Value) [ng/mL] |
|---|---|---|
| Monosaccharide | Tagatose | 100 |
| Disaccharide | Cellobiose | 114 |
| | Lactulose | |
| | Trehalose | |
| | Sialyllactose | |
| Trisaccharide | Kestose | 186 |
| | Lactosucrose | |
| | Raffinose | |
| | Fucosyllactose | |
| Tetrasaccharide | Stachyose | 141 |
| | Nystose | |
| Mixed Sugar | Fructooligosaccharide | 149 |
| | Galactooligosaccharide | |
| Sugar Alcohol | Reduced Isomaltulose | 39 |
| | Lactitol | |
| | Sorbitol | |
| | Mannitol | |
| | Xylitol | |
| Dietary Fiber | Inulin | 70 |

As shown in Fig. 2, NMN was detected in the evaluation media to which each carbohydrate was added, unlike the evaluation medium to which no carbohydrate was added (DW). In particular, kestose, cellobiose, fructooligosaccharides, lactosucrose, and raffinose had higher NMN concentrations than glucose and sucrose, which are common sugars used in cultures (however, these are decomposed and absorbed in the upper digestive tract in *vivo*). As shown in Table 2, the average NMN concentration for each carbohydrate classification was highest for trisaccharides, followed by mixed sugars (mixed sugars of tri- to pentasaccharides), tetrasaccharides, and disaccharides in descending order. However, as shown in Fig. 2, even among disaccharides, sialyllactose, which is a sialylated modified sugar, had a relatively low NMN concentration. Furthermore, even among trisaccharides, fucosyllactose, which contains fucose in its constituent sugars, had a relatively low NMN concentration.

### <Test Example 3> Evaluation of Improvement of NMN Production in Mouse Intestine

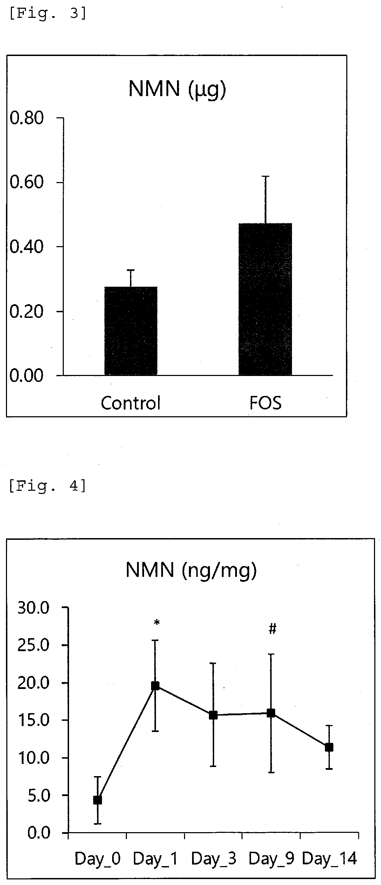

Among the carbohydrates for which excellent NMN production-improving effects were confirmed in Test Example 2, fructooligosaccharides (FOS, a mixture of 1-kestose, nystose, and 1-fructofuranosyl-D-nystose, manufactured by Meiji Food Materia Co., Ltd., product name: MeiOligo P) were used to evaluate the effect of improving NMN production in the mouse intestine (large intestine). First, seven-week-old male ICR mice (manufactured by Japan SLC, Inc.) were divided into two groups of eight individuals each, one group was allowed free access to a 5% FOS aqueous solution (FOS group) and the other group was allowed free access to water (Control group), and each group was bred for 10 days. After the breeding period, the cecal contents were collected after fasting for 3 hours, and the weight thereof was measured. 20 mg of the collected cecal contents was weighed into a microtube, mixed with methanol so that the final concentration was 80 v/v%, and allowed to stand on ice for 30 minutes. Next, the supernatant obtained by centrifuging this was transferred to another microtube, 80 v/v% methanol was added to the pellet and mixed, and the supernatant obtained by centrifugation again was mixed with the supernatant previously transferred to another microtube. Next, the mixed supernatant was dried using a centrifugal evaporator, the obtained dried product was dissolved in an ammonium acetate solution, filtered through a 0.22 µm filter, and then the NMN concentration in the filtrate was measured by liquid chromatograph mass spectrometer (LC-MS/MS) by the method described in Test Example 1 to calculate the NMN concentration in the cecal contents. Further, the amount (total (µg)) obtained by multiplying the measured NMN concentration by the weight of the cecal contents was calculated, and the average value of total in each group was calculated. The results are shown in Fig. 3.

As shown in Fig. 3, the amount of NMN in the cecal contents was higher in the FOS group given fructooligosaccharides than in the Control group given water; a significance test between the Control group and the FOS group was performed by t-test, and p < 0.01 was obtained, confirming a significant difference between the two groups. Therefore, it was confirmed that fructooligosaccharides increase the amount of NMN produced by the gut microbiota *in vivo,* and it was suggested that at least one selected from the group consisting of kestose, nystose, and fructofuranosylnystose comprised therein also has the same effect.

### <Test Example 4> Evaluation of Improvement of NMN Production in the Intestine of Human Flora Mice

Using kestose (manufactured by Meiji Food Materia Co., Ltd.), the effect of improving NMN production in the intestine (large intestine) of human flora mice, which were germ-free mice transplanted with human gut microbiota, was evaluated. First, human feces were orally administered to eight-week-old male germ-free mice (C57BL/6N, manufactured by CLEA Japan, Inc., 3 individuals), acclimatized for 3 weeks, and then feces were collected (Day 0). During this period, water was given ad libitum. After fecal collection, the drinking water was changed from water to a 5% kestose aqueous solution, which was given ad libitum, and the mice were bred for 14 days. Feces were collected 1, 3, 9, and 14 days after the start of kestose administration (Day 1, 3, 9, and 14) . 20 mg of the feces collected on each fecal collection day (Day) was weighed into a microtube, mixed with methanol so that the final concentration was 80 v/v%, and allowed to stand on ice for 30 minutes. Next, the supernatant obtained by centrifuging this was transferred to another microtube, 80 v/v% methanol was added to the pellet and mixed, and the supernatant obtained by centrifugation again was mixed with the supernatant previously transferred to another microtube. Next, the mixed supernatant was dried using a centrifugal evaporator, the obtained dried product was dissolved in an ammonium acetate solution, filtered through a 0.22 µm filter, and then the NMN concentration in the filtrate was measured by liquid chromatograph mass spectrometer (LC-MS/MS) by the method described in Test Example 1 to calculate the NMN concentration (ng/mL) in the feces. The average values of the NMN concentrations of the three individuals on each fecal collection day are shown in Fig. 4.

As shown in Fig. 4, the NMN concentration in the feces was higher on Day 1, 3, and 9 during the kestose administration period than on Day 0 before the kestose administration period, and on Day 1 and 9, p < 0.10 (# in Fig. 4) or p < 0.05 (* in Fig. 4) was obtained by Dunnett's test. Therefore, it was confirmed that kestose increases the amount of NMN produced by human gut microbiota *in vivo.*

### [Industrial Applicability]

As described above, according to the present invention, it is possible to provide a composition having an effect of improving nicotinamide mononucleotide (NMN) production by the gut microbiota. Further, the composition of the present invention can be provided as a food and/or drink composition, and therefore, it is easy to ingest and easy to make ingestion a habit. Furthermore, the composition of the present invention can be used for improving NAD concentration *in vivo,* preventing or ameliorating age-related diseases, and preventing or ameliorating constipation and inflammatory bowel disease by increasing the amount of NMN produced by the gut microbiota.

## Claims

1. A composition for improving nicotinamide mononucleotide (NMN) production, comprising at least one carbohydrate selected from the group consisting of monosaccharides (excluding glucose and xylose), disaccharides, trisaccharides, tetrasaccharides, pentasaccharides, sugar alcohols, and dietary fiber as an active ingredient.

2. The composition according to claim 1, comprising a monosaccharide as the carbohydrate, wherein the monosaccharide is tagatose.

3. The composition according to claim 1, comprising a disaccharide as the carbohydrate, wherein the disaccharide is at least one selected from the group consisting of cellobiose, lactulose, trehalose, and sialyllactose.

4. The composition according to claim 1, comprising a trisaccharide as the carbohydrate, wherein the trisaccharide is at least one selected from the group consisting of kestose, lactosucrose, raffinose, and fucosyllactose.

5. The composition according to claim 1, comprising a tetrasaccharide as the carbohydrate, wherein the tetrasaccharide is at least one selected from the group consisting of stachyose and nystose.

6. The composition according to claim 1, comprising a pentasaccharide as the carbohydrate, wherein the pentasaccharide is fructofuranosylnystose.

7. The composition according to claim 1, comprising a sugar alcohol as the carbohydrate, wherein the sugar alcohol is at least one selected from the group consisting of reduced isomaltulose, lactitol, sorbitol, mannitol, and xylitol.

8. The composition according to claim 1, comprising dietary fiber as the carbohydrate, wherein the dietary fiber is inulin.

9. The composition according to any one of claims 1 to 8, which is an oral composition or an enteral composition.

10. The composition according to any one of claims 1 to 8, which is a food and/or drink composition, a pharmaceutical composition, or a quasi-pharmaceutical composition.

11. The composition according to any one of claims 1 to 8, which is a composition for preventing or ameliorating constipation.

12. The composition according to claim 11, wherein the dose to humans is 10 to 1000 mg of the carbohydrate per kg body weight per day.

13. The composition according to any one of claims 1 to 8, which is a composition for preventing or ameliorating inflammatory bowel disease.

14. The composition according to claim 13, wherein the dose to humans is 10 to 1000 mg of the carbohydrate per kg body weight per day.

15. The composition according to any one of claims 1 to 8, which is a composition for preventing or ameliorating age-related diseases.

16. The composition according to claim 15, wherein the dose to humans is 10 to 1000 mg of the carbohydrate per kg body weight per day.
